# EUROPEAN PATENT APPLICATION

(11) **EP 3 797 786 A1**
(43) Date of publication of application: **31.03.2021**
(21) Application number: 20204479.8
(22) Date of filing: 14.10.2015
(51) Int. Cl.: A61K 38/10

(54) **COMPOSITIONS AND METHODS FOR TREATMENT OF DISEASES**

(30) Priority: 15.10.2014 IE S20140274; 13.01.2015 IE S20150034; 18.06.2015 IE S20150183; 17.08.2015 IE S20150270
(62) Divisional of application: 15813581.4
(71) Applicant: Byrock Technologies Limited, Dublin 2 (IE)
(72) Inventor: PRENDERGAST, Patrick T., Dublin 2 (IE)
(74) Representative: FRKelly

(57) **Abstract**

A method for treating or preventing laminitis is provided comprising administering to an ungulate a therapeutically or prophylactically effective amount of an anti-hemorrhagic peptide.

## Description

### Field of the Invention

The present invention relates to compositions comprising protease inhibitors from camelid and methods for using same in the treatment and prevention of diseases in ungulates, in particular, for the treatment and prevention of equine laminitis.

### Background of the Invention

Laminitis is an inflammation of the hoof corium. It occurs especially in horses, but is also found in other ungulates. Since the second half of the 19^{th} century, several essays on laminitis appear in monographs, scientific papers and textbooks. In the 19^{th} and 20^{th} century, scientific and technical progress increased available knowledge on the character and the pathogenesis of laminitis. New possibilities concerning diagnostics and therapy were established. However, up until today laminitis remains one of the most important diseases, especially for horses, and even with new knowledge and therapies a cure of laminitis has remained elusive.

For bovinae, the view concerning the appearance and impact of laminitis changed in the last decades. Today, laminitis is regarded as the most important claw disease (Lischer CJ, Ossent P: Pathogenesis of sole lesions attributed to laminitis in cattle. Proceedings of the 12th International Symposium on Lameness in Ruminants, Orlando, Florida, January 9-13, 2002a, 82-89). It also works as an underlying disease for many claw disorders. Laminitis, and the accompanying local trauma, has been made responsible for the development of sole ulcers (A Rusterholz: The specific traumatic sole ulcer of claws in cattle, Schw Arch Tierheilk, 1920; J.J. Vermunt, P.R. Greenough: Sole haemorrhages in dairy heifers managed under different underfoot and environmental conditions, British Veterinary Journal, Volume 152, Issue 1, January 1996, Pages 57-73). The immune system of, for example, the dairy cow is very vulnerable especially around the time of birth (35 days before calving to 70 days after calving) because of massive changes in its metabolism. Because of diagnostic uncertainties and therefore limited possibilities in practice, basic data for the frequency of the appearance of laminitis are missing.

Concerning the pathogenesis of laminitis, the contacts of the corium lamellas become loose from the epidermis lamellas, which physiologically grip into each other like a zipper. Local edema formation and swelling, caused through higher outlet of tissue liquid and blood cells, results in high pressure between the coffin bone and the hoof wall. As there is no possibility of expansion, the high pressure causes heavy pain in that area for the animal. In the heavy gradient, this is called chronic laminitis after only 48 hours. Further effects of the loosening of the contacts are the sinking and rotation of the coffin bone in the capsule and a rotation of the coffin joint up to a possible breakthrough of the tip of the coffin bone through the sole with hoof loss, which is a complete detachment of the hoof capsule.

The most common cause of laminitis is laminitis from carbohydrate rich food, but there are also laminitis after birth, laminitis after poisoning (e.g. through consuming poisonous mushrooms), laminitis after too much exposure to very hard grounds, or laminitis after medicaments (e.g. cortisone). Furthermore, there are also other clinical pictures which cause laminitis, e.g. colic, enteritis, lumbago, thyroid diseases and Cushing's syndrome. Also discussed as causes of laminitis are types of housing of the animals and their psyche (e.g. stress). Other known causes include allergies. On one hand, toxins that can be generated through too high a protein supply, too much starch or different kinds of sugars in high concentrations (e.g. fructans) can induce laminitis. On the other hand, laminitis can be induced by consuming poisonous plants, pesticides, fertilisers or overdoses of medicaments. More frequent factors that can induce laminitis include unnatural preserving agents and additives of nutrients that are not of the natural habitat of the animals in question. In relation to bovinae, previous systemic diseases like rumen acidaemia and endometritis are known triggers for laminitis, which causes functional and morphological changes in the capsule.

In addition to bovinae, laminitis also occurs commonly in other ungulates from families like equidae, suidae, deer, ovis and capra.

Metalloproteinases and serine proteases are naturally occurring enzymes present in many tissues of the equine body and in mammals in general. These enzymes act to degrade proteins, normally in a controlled and specific manner. To prevent the uncontrolled destruction of target proteins and tissue such as the hoof, the activity of these proteolytic enzymes is modulated by inhibitor serum peptides normally present under healthy conditions wherein the combined and balanced actions of proteases and inhibitors act to control the level of biologically active and structurally important proteins of the body, thereby regulating many important physiological processes and maintaining structural integrity.

One important group of proteinases is the metalloproteinases (also known as metalloproteases or MMPs). These enzymes are characterised by their requirement for the presence of a metal ion in order to catalyse proteolysis. Approximately 17 different metalloproteinases have been identified and/or cloned which share significant sequence homology. The metalloproteinase family can be subdivided into five groups according to their structural and functional properties: (i) the collagenases (metalloproteinases-1, 8 and 13); (ii) gelatinases A and B (metalloproteinase-2 and metalloproteinase-9); (iii) stromelysins 1 and 2 (metalloproteinase-3 and metalloproteinase-10); (iv) matrilysin (MMP-7), enamelysin (MMP-20), macrophage metalloelastase (MMP-12) and MMP-19 (making up the classical metalloproteinases); and (v) membrane-type metalloproteinases (MT-MMP-1 to 4, stromelysin-3 and MMP-11). These metalloproteinases share a common multi-domain structure, but are glycosylated to different extents and at different sites. According to sequence alignment, the assembly of these domains might have been an early evolutionary event, followed by diversification.

Collectively, metalloproteinases can degrade all the major components of the extracellular matrix (ECM). The homeostasis of the ECM is controlled by a delicate balance between the synthesis of ECM proteins, production of ECM-degrading extracellular matrix metalloproteinases and the presence of metalloproteinase inhibitors.

One family of metalloproteinases inhibitor peptides is the tissue inhibitors of metalloproteinases (TIMPs). The TIMP family is comprised of at least four distinct members (TIMP-1 to 4) that possess 12 conserved cysteine residues and express metalloproteinase inhibitory activity by forming non-covalent complexes with metalloproteinases enzymes. Specifically TIMPs bind to the highly conserved active zinc-binding site of metalloproteinases in a 1:1 stoichiometry, but can also bind at other domains of metalloproteinase-2

WO 2010126544 describes use of mast cell stabilizers to prevent, treat or mitigate the severity of laminitis. US 20140144109 discloses a boot for treating laminitis in horses wherein the boot has a hoof casing for snugly receiving and supporting a horny hoof wall of the laminitic hoof and a sole pivotally attached to the hoof casing such that the laminitic hoof may pivot with respect to the sole while the sole is planted on the ground, thereby reducing stress on the inflamed laminae. EP 2497475 describes use of specific antiplatelet drugs for the treatment and/or prevention of laminitis. However, the prior art in this field has not been able to effectively prevent or cure the disease.

### Summary of the Invention

According to a first aspect the present invention, there is provided a method for treating or preventing a disorder associated with undesirable protease activity in a subject in need thereof, the method comprising administering a therapeutically or prophylactically effective amount of a camelid protease inhibitor to the subject.

The undesirable protease activity may be undesirable activity associated with one or more proteases selected from metalloproteinases and serine proteases. The undesirable protease activity typically refers to increased protease activity.

The disorder associated with undesirable protease activity typically refers to laminitis, but may also include other veterinary diseases of subjects where undesirable protease activity, in particular, elevated protease activity, is responsible for the disorder. The laminitis may be chronic or acute laminitis. The disorder may include shin or ocular infections of subjects. The disorder may also include wounds. The disorder may also be a gastrointestinal injury, disease or ulcer. The disorder may be selected from the group consisting of equine chronic lung disease, equine osteoarthritis disease, equine septic joint disease, equine colic, equine chronic obstructive pulmonary disease, equine joint disease, equine ulcerative colitis, equine Crohns disease and equine inflammatory bowel disease.

The subject may be an ungulate, in particular a hoofed ungulate. In particular, the subject may be selected from the group consisting of equidae, bovinae, suidae, deer, ovis and capra. Typically the subject is a horse.

The camelid protease inhibitor may be an inhibitor of one or more metalloproteinases (MMPS) and/or one or more serine proteases. In particular, the camelid protease inhibitor may be an inhibitor of equine metalloproteinases and equine serine proteases. Typically the inhibitor is a tissue inhibitor of a metalloproteinase (TIMP). Typically the inhibitor inhibits elastase. Typically the inhibitor inhibits disintegrin and metalloproteinase enzymes, particularly ADAM-TS4/5 (also termed aggrecanase 1/2), MMP-2 and MMP-9.

The inhibitor may be obtained or derived directly or indirectly from blood (serum or plasma) of a camelid. In particular, the inhibitor may be isolated or purified from blood (serum or plasma) of a camelid. The inhibitor may be naturally occurring in camelid blood (serum or plasma). The inhibitor may also be produced recombinantly or synthetically based on the isolated or purified inhibitor. Alternatively, the inhibitor may be utilised incorporated in camelid plasma.

Alternatively or additionally, the inhibitor may be generated following a vaccination program in camelid. In particular, the inhibitor may be generated by inoculating camelid with enzymes known to cause laminitis, typically with purified metalloproteinase enzymes and serine proteases, in particular equine MMPs and serine proteases. The inhibitor may also be generated by inoculating camelid with snake venom metalloproteinases (SVMPs), for example MMPs from *Bothrops jararaca.* The MMPs may be incorporated in a suitable adjuvant. The inhibitor may be found in hyperimmune camelid plasma generated to SVMPs. The inventor has identified that SVMPs are very effective antigens in eliciting a protective homodimer antibody response in vaccinated camelid and these can also be generated by recombinant methods.

Typically the inhibitor is a peptide, more typically the inhibitor may be a homodimer antibody or an antigen binding fragment of same, in particular, a single domain antigen binding fragment. These may be generated by immunizing camelid as described above. These single domain proteolytic enzyme inhibitory antibodies and fragments of same may also be manufactured recombinantly utilizing techniques currently available in the literature. The antibody may be directed against the active enzymatic site of a protease, such as a metalloproteinase enzyme. In particular, the inhibitor may be an isolated variable domain of such an antibody (VHH domain). The VHH domain may be obtained by a method comprising the steps of: (a) immunising a camelid with a selected metalloproteinase enzyme as antigen; (b) isolating peripheral lymphocytes of the immunized camelid, obtaining the total RNA and synthesizing the corresponding cDNAs; (c) constructing a library of cDNA fragments encoding VHH domains; (d) transcribing the VHH domain-encoding cDNAs obtained in step (c) to mRNA using PCR, converting the mRNA to ribosome display format, and selecting the VHH domain by ribosome display; and (e) expressing the VHH domain in a vector.

The inhibitor may comprise both naturally occurring inhibitors from camelid blood and inhibitors generated by vaccination of camelid as described above.

In certain embodiments the camelid protease inhibitor is administered with an anti-hemorrhagic peptide. The anti-hemorrhagic peptide may be obtained from opossum serum or cotton rat, or may be a recombinant form thereof. In certain embodiments, the anti-hemorrhagic peptide is selected from the following or a combination thereof:
Phe-Leu-His = Peptide 1
Trp-Leu-Phe = Peptide 2
Trp-Leu-Try = Peptide 3
Trp-Leu-Arg = Peptide 4
Trp-Leu-His = Peptide 5
Phe-Leu-Phe = Peptide 6
Phe-Leu-Try = Peptide 7
Phe-Leu-Arg = Peptide 8

In certain embodiments, the peptide is coupled to hydroxamate.

In certain embodiments, the peptide comprises, consists of or consists essentially of one of the following sequences:
Leu-Lys-Ala-Met-Asp-Pro-Thr-Pro-Pro-Leu-Trp-Ile-Lys-Thr-Glu (SEQ ID NO:1)
Leu-Lys-Ala-Met-Asp-Pro-Thr-Pro-Pro-Leu (SEQ ID NO:2).

In certain embodiments, the peptide has at least 85%, 90%, 95%, 96%, 97%, 98% or 99% sequence identity with SEQ ID NO:1 or SEQ ID NO:2.

The anti-hemorrhagic peptide may be administered simultaneously with, sequentially with or separately to the camelid protease inhibitor.

Cell-in-a-box^{RTM} technology may be utilized to implant camelid B-cells secreting inhibitory homodimer antibodies as described above in the subject. Alternatively, cell-in-a-box^{RTM} technology may be utilised to implant cells secreting recombinant single-domain antibody fragments capable of inhibiting metalloprotease enzymes and other factors involved in the pathogenesis of laminitis.

The inhibitor may be administered after exposure to a predisposing event that commonly results in laminitis, such as carbohydrate rich food, birth, poisoning (e.g. through consuming poisonous mushrooms), exposure to very hard grounds, medicaments (e.g. cortisone), colic, enteritis, lumbago, thyroid diseases, Cushing's syndrome, stress, allergies, unnatural preserving agents, additives of nutrients that are not of the natural habitat of the subject in question and systemic diseases like rumen acidaemia and endometritis. The subject may be suffering from laminitis prior to administration of the inhibitor or administration may be prophylactic.

The above aspect of the invention is based on the unexpected finding by the inventor that camelid serum/plasma contains effective inhibitory amounts of equine metalloproteinase and general serine protease inhibitor peptides. These are similar in amino acid sequence and function to anti-hemorrhagic peptides isolated from opossum and other mammals having these serum components present in their serum - accordingly, in certain embodiments the camelid protease inhibitor comprises one or more of the amino acid sequences shown below, or a combination thereof. Camelid surprisingly demonstrate serum anti-hemorrhagic ability in large concentrations unlike any other ruminant serum tested. Similar inhibitors have not been found or isolated from other domestic animal serum tested, including bovine, caprine and equine species. The present invention identifies camelid serum/plasma as a plentiful source of metalloproteinase inhibitor peptides and serine protease inhibitory proteins. It has also been found that inoculating camelids with purified equine metalloproteinase enzymes and serine proteases and adjuvant results in the generation of enzyme inhibitory homodimer antibodies in the inoculated camelid and these antibodies have the ability to inhibit metalloprotease enzymatic activity and elastase. These antibodies may be used to inhibit these enzymes in the laminitis hoof leading to rapid healing or total prevention of laminitis.

According to a second aspect of the present invention, there is provided a composition comprising a camelid protease inhibitor.

The inhibitor may be an inhibitor as described above in relation to the first aspect of the invention. In particular, the composition may comprise a camelid protease inhibitor purified or isolated from camelid blood, or a recombinant form thereof.

In certain embodiments, the composition comprises a peptide comprising, consisting of or consisting essentially of SEQ ID NO:1 or SEQ ID NO:2. In certain embodiments, the peptide has at least 85%, 90%, 95%, 96%, 97%, 98% or 99% sequence identity with SEQ ID NO:1 or SEQ ID NO:2.

A further aspect of the inventions relate to a camelid protease inhibitor for use as a medicament. In particular, the invention relates to a camelid protease inhibitor for use in the treatment and prophylaxis of a disorder associated with undesirable protease activity in a subject in need thereof. Also provided is use of a camelid protease inhibitor in the preparation of a medicament for the treatment and prophylaxis of a disorder associated with undesirable protease activity in a subject in need thereof. Further provided is a pharmaceutical composition comprising a camelid protease inhibitor and one or more pharmaceutically acceptable excipients.

According to a further aspect of the present invention, there is provided a method for generating antibodies comprising inoculating camelid with one or more proteases selected from the group consisting of equine metalloproteinase enzymes, equine serine proteases and snake venom metalloproteinases (SVMP). The proteases may be purified prior to inoculation and may be administered with an adjuvant.

For example, a VHH domain may be obtained by a method comprising the steps of: (a) immunizing a camelid with a selected metalloproteinase enzyme as antigen; (b) isolating peripheral lymphocytes of the immunized camelid, obtaining the total RNA and synthesizing the corresponding cDNAs; (c) constructing a library of cDNA fragments encoding VHH domains; (d) transcribing the VHH domain-encoding cDNAs obtained in step (c) to mRNA using PCR, converting the mRNA to ribosome display format, and selecting the VHH domain by ribosome display; and (e) expressing the VHH domain in a vector.

The invention extends to antibodies and antigen binding fragments obtained using the above methods and to vectors comprising the VHH domain and hosts expressing same.

According to a further aspect of the present invention there is provided a method for at least partially purifying or enriching a camelid protease inhibitor, the method comprising steps of subjecting camelid serum and/or camelid plasma to one or more treatment steps selected from the group consisting of centrifugation, micro- filtration, ultra-filtration, ion-exchange chromatography, molecular sieve chromatography, affinity chromatography, reverse-phase high performance liquid chromatography and transient acidification.

Embodiments described above in relation to the first aspect of the invention apply mutatis mutandis to these further aspects of the invention. In particular, the inhibitor may be an inhibitor as described above in relation to the first aspect of the invention.

According to a further aspect of the present invention, there is provided a method for treating or preventing a disorder associated with undesirable protease activity in a subject in need thereof, the method comprising administering a therapeutically or prophylactically effective amount of an anti-hemorrhagic peptide.

In certain embodiments, the anti-hemorrhagic peptide is a camelid protease inhibitor, for example as described above. In certain embodiments the anti-hemorrhagic peptide is obtained from opossum serum or cotton rat, or is a recombinant form thereof. In certain embodiments, the anti-hemorrhagic peptide is selected from the following or a combination thereof:
Phe-Leu-His = Peptide 1
Trp-Leu-Phe = Peptide 2
Trp-Leu-Try = Peptide 3
Trp-Leu-Arg = Peptide 4
Trp-Leu-His = Peptide 5
Phe-Leu-Phe = Peptide 6
Phe-Leu-Try = Peptide 7
Phe-Leu-Arg = Peptide 8

In certain embodiments, the peptide is coupled to hydroxamate.

In certain embodiments, the peptide comprises, consists of or consists essentially of one of the following sequences:
Leu-Lys-Ala-Met-Asp-Pro-Thr-Pro-Pro-Leu-Trp-Ile-Lys-Thr-Glu (SEQ ID NO:1)
Leu-Lys-Ala-Met-Asp-Pro-Thr-Pro-Pro-Leu (SEQ ID NO:2).

In certain embodiments, the peptide has at least 85%, 90%, 95%, 96%, 97%, 98% or 99% sequence identity with SEQ ID NO:1 or SEQ ID NO:2.

Embodiments described above in relation to the first aspect of the invention apply mutatis mutandis to these further aspects of the invention.

### Detailed description of the invention

The inventor has demonstrated that naturally occurring peptides in camelid serum/plasma are a useful source of protease enzyme inhibitors, in particular, equine metalloproteinase enzyme inhibitors. These are not present in such elevated levels or with such broad spectrum protease inhibition in other ruminants and the unexpected finding of these inhibitors in camelid serum/plasma provides a plentiful, renewable source of equine metalloproteinase enzyme inhibitor peptides and non-metal protease enzyme inhibitor peptides. The peptide inhibitor isolated from camelid serum and/or plasma is capable of inhibiting membrane type matrix metalloproteinases and non-metal bearing general protease enzymes such as elastase. The inventor has further identified that homodimer antibodies for use in the treatment of laminitis may be generated following a vaccination program in camelid using purified equine metalloproteinase enzymes and serine proteases or snake venom metalloproteinases (SVMPs). These antibodies have demonstrated efficacy in the treatment and prevention of laminitis as shown in a study carried out on nine horses. More specifically these enzyme inhibitory peptides/homodimer antibodies and their fragments are active against equine metalloproteinases and other equine serine proteases enzymes.

The inhibitor may be administered alone, but will preferably be administered as a pharmaceutical composition, which will generally comprise a suitable pharmaceutical excipient, diluent or carrier selected depended on the intended route of administration.

The inhibitor may be provided at a concentration ranging from about 0.01 µg/ml to about 100mg/ml in the formulation prepared for the application of this invention. Typically the inhibitor is present at a concentration ranging from about 0.1 µg/ml to about 1000µg/ml. More typically the inhibitor is present at a concentration ranging from about 1 µg/ml to 500µg/ml. Even more typically, the inhibitor is present at a concentration of about 11 µg/ml or about 45µg/ml or about 50µg/ml, as quantified by a fluorescence-quenching substrate assay.

Pharmaceutical compositions according to the present invention may be adapted for administration in any suitable manner. The composition may be adapted for internal or topical administration. The composition may be in an oral, injectable, topical or suppository form or formulated in a gel to make application to wound surfaces more convenient. Preferred delivery routes include intravenous, dermal, intravaginal, respirator, and gastrointestinal.

Methods and pharmaceutical carriers for preparation of pharmaceutical compositions, including compositions for topical administration are well known in the art, as set out in textbooks such as Remington's Pharmaceutical Sciences, 18th Edition, Mack Publishing Company, Easton, Pennsylvania, USA.

Compositions of the present invention may be formulated so that they are suitable for oral administration. The compositions may be presented as discrete units such as capsules, sachets or tablets or in bandages each containing a predetermined amount of the inhibitor, or as a powder, granules or gel, as a solution or a suspension in an aqueous or non-aqueous liquid, as a mouthwash or as an oil-in-water liquid emulsion or a water-in-oil liquid emulsion. The active ingredient may also be presented as a bolus, electuary or paste.

In addition to the ingredients particularly mentioned above, the compositions of this invention may include other agents conventional in the art having regard to the type of therapeutic in question, for example, those suitable for oral administration may include such further agents as sweeteners, thickeners and flavoring agents.

The compositions of the present invention may include a carrier selected from the group consisting of a synthetic or biological polymer, glycosaminoglycan, or extracellular matrix molecule including fibrin, collagen, gelatin, a synthetic polymer, agarose, an alginate, methylcellulose, hyaluronic acid, a hydrocolloid, an alginate, saline solution, powder, ointment, salve or incorporated or impregnated into a dressing (absorbable and non-absorbable), a transdermal patch or releasable dressing associated with gauze, a bandage, suture, plaster, staple, prosthetic device, screw or plate (biodegradable or non- biodegradable), toothpaste, gum or resin for chewing, mouth wash or gel. The skilled artisan will be familiar with the appropriate carrier to use depending on the route or means for administration.

The composition may have at least one further active ingredient selected from the group consisting of antibiotics, anti-inflammatories, antiseptics and other agents, e.g. anesthetics. The compositions described herein may have other molecules associated therewith to aid releasability, stability, solubility, activity and/or association with wound healing, including carriers, solubilizing agents, and growth factors. The composition may also include one or more secondary therapeutic agents for treatment of the disorder in question, such as laminitis.

The inhibitor or camelid plasma may be injected intravenously into the ungulate, for example, the hoof. The ungulate may be suspected of developing or have the sequela of laminitis at the time of administration. Administration may prevent laminar detachment. The inhibitor or plasma may be administered into a flexor digitorum profundus muscle or into a blood supply of a limb of the ungulate.

The composition may be applied directly to wounds in a biologically acceptable carrier to ensure sustained release at sufficient concentration in the wound environment. In treating a wound, the inhibitor may be associated with a wound support, gel or suitable solution. The wound to be supported may be a wound created by surgery, or the result of accident or other injury. The composition or inhibitor may be present on the surface of the wound support or may be impregnated in the wound support/gel and released therefrom.

The wound to be treated according to this invention may be an ulcer caused by pressure, vascular disease, diabetes, autoimmune disease, sickle cell diseases or as a result of surgery; therapeutically induced; associated with disorders of the central nervous system, and resulting from any exfoliative disease of the skin; associated with either local or systemic infection or a corneal injury to the eye; a pathological wound; a traumatic or accidental wound; or a burn.

Typically the concentration of the inhibitor is from about 0.1 ng/ml to about 10µg/ml of fluid in the local environment at the wound or disease site. More typically the concentration of the inhibitor is from about 1 ng/ml to about 1 µg/ml of fluid in the local environment at the wound site.

The present invention also provides a method for preventing, ameliorating or treating a condition associated with a gastrointestinal injury, disease or ulcer, the method including administering to the animal in need thereof an effective amount of composition as described herein. In a preferred method the concentration of the inhibitor (anti-hemorrhagic peptide) present in the medication should range from about 0.1 µg/ml to about 10mg / ml.

The composition may be administered at any appropriate time including prior to, during or after the disorder has become evident. Typically two or more doses may be administered over time.

The disorder can be a dental or oral wound; peptic ulceration of the duodenum, stomach or esophagus; inflammatory bowel disease; an ulcer associated with stress conditions; damage to the lining of the alimentary tract; inadequate gut function or damage to the gut associated with prematurity; a diarrheal condition; a food intolerance; a cancer of the gastrointestinal tract; surgically induced damage to the gut; damage due to esophageal reflux; a condition associated with loss of gut barrier function; a congenital condition resulting in inadequate gastrointestinal function or damage; or an autoimmune disease that affects the gut.

For all methods of treatment described herein the daily dosage can be routinely determined by the attending physician or veterinarian. Generally the dosage will vary according to the age, weight, and response of the individual patient, as well as the severity of the patient's symptoms. In general a suitable dose of the inhibitor of the invention will be in the range of about 0.1 µg to about 100mg per kilogram body weight of the recipient per day, preferably in the range of about 1 µg to about 50mg per kilogram body weight per day. However, the dose will also depend on the formulation and purity of the camelid serum and or plasma used and the concentration of inhibitor present.

### Definitions

As used herein, "camelid protease inhibitor" refers to a protease inhibitor which is obtainable from camelid. The inhibitor may be purified from camelid blood or may be a recombinant or synthetic version of a protease inhibitor purified from camelid blood. For example, the inhibitor may be manufactured recombinantly using *E. coli* or using Cell-in-a-box^{RTM} technology. The inhibitor may also be an antibody obtained following immunization of a camelid as described above.

The term "camelid" refers to the group of even-toed ungulate mammals which form the family Camelidae. These include camels, such as Camelus bactrianus and Camelus dromderius, and llama, such as Lama Paccos, Lama Glama and Lama Vicugna.

As used herein the term "metalloproteinase" includes proteases that proteolytically degrade a component of the extracellular matrix. The term metalloproteinases includes, but is not limited to (i) the collagenases (metalloproteinases-1, 8 and 13); (ii) gelatinases A and B (metalloproteinase-2 and metalloproteinase-9); (iii) stromelysins 1 and 2 (metalloproteinases-3 and 10); (iv) matrilysin (MMP-7), enamelysin (MMP- 20), macrophage metalloelastase (MMP12) and MMP-19 (making up the classical metalloproteinases) and (v) membrane-type metalloproteinases (MT-MMP-1 to 4, stromelysin-3 and MMP-11). Metalloproteinase 2 is also known as gelatinase A. Metalloproteinase 2 is a proteolytic enzyme having a molecular weight of 72kDa which catalyses the degradation of collagen type IV by acting on the peptide bonds. Metalloproteinase 9 is also known as gelatinase B. Metalloproteinase 9 is a proteolytic enzyme having a molecular weight of 92kDa which catalyses the degradation of collagen type IV by acting on the peptide bonds.

As used herein the term "tissue inhibitor of a metalloproteinase" includes, but is not limited to, polypeptides isolated from camelid blood or opossum serum or synthetically or recombinantly produced which regulate the activity of equine metalloproteinases which includes TIMP-1, TIMP-2, TIMP-3 and TIMP-4. The TIMP family is comprised of at least four distinct members (TIMP-1 to 4) that possess 12 conserved cysteine residues and express metalloproteinase inhibitory activity by forming non-covalent complexes with metalloproteinases. Specifically TIMPs bind to the highly conserved active zinc-binding site of the metalloproteinases in a 1:1 stoichiometry, but can also bind at other domains of metalloproteinase-2 and metalloproteinase-9.

As used herein the term "wound support" includes any means which is used to support or secure a wound and includes a surgical securing means. The term includes plasters, dressings, sutures, staples and the like.

The term "treatment" is used herein to refer to any regime that can benefit a subject. References herein to "therapeutic" and "prophylactic" treatment are to be considered in their broadest context. The term "therapeutic" does not necessarily imply that a subject is treated until total recovery. Similarly, "prophylactic" does not necessarily mean that the subject will not eventually contract a disease condition. Accordingly, therapeutic and prophylactic treatment includes amelioration of the symptoms of a particular condition or preventing or otherwise reducing the risk of developing a particular condition.

A "therapeutically effective amount" or "prophylactically effective amount" means the amount required at least partly to attain the desired effect, i.e. to alleviate or remove the symptoms of undesirable protease enzymatic activity or alternatively to delay the onset of, inhibit the progression of, or halt altogether, the onset or progression of the undesirable protease activity, or to reduce metalloproteinase / protease activity. Preferably the term "therapeutically effective amount" as used herein means amount sufficient to elicit a statistically significant response at a 95% confidence level. Such amounts will depend, of course, on the particular condition being treated, the severity of the condition, and individual subject parameters, including age, physical condition, size, weight and other concurrent treatment, and will be at the discretion of the attending veterinary person. These factors are well known to those of ordinary skill in the art, and can be addressed with no more than routine experimentation. It is generally preferred that a minimum effective dose be determined according to sound veterinary judgment.

An "antibody" is an immunoglobulin, whether natural, partly or wholly synthetically produced. The term also covers any polypeptide, protein or peptide having a binding domain that is, or is homologous to, an antibody binding domain. These can be derived from natural sources, or they may be partly or wholly synthetically produced. The invention extends to the use of antigen binding fragments of camelid antibodies, chimeric antibodies and diabodies as well as polypeptides that contain at least a portion of an immunoglobulin that is sufficient to confer specific antigen binding to the polypeptide. As antibodies can be modified in a number of ways, the term "antibody" should be construed as covering any binding member or substance having a binding domain with the required specificity. The antibody of the invention may be a monoclonal antibody, or a fragment, derivative, functional equivalent or homologue thereof. The term includes any polypeptide comprising an immunoglobulin binding domain, whether natural or wholly or partially synthetic.

In certain embodiments where an anti-hemorrhagic peptide is administered the anti-hemorrhagic peptide may be selected from the following or any combination thereof:
1. Sequence Description (as described in US5,576,297):
   Leu-Lys-Ala-Met-Asp-Pro-Thr-Pro-Pro-Leu-Trp-Ile-Lys-Thr-Glu (SEQ ID NO:1)
   Leu-Lys-Ala-Met-Asp-Pro-Thr-Pro-Pro-Leu (SEQ ID NO:2)
2. Collection of Sequences (Villalta-Romero et al., ACS Med. Chem. Lett. 2012, 3, 540-543):
   Phe-Leu-His = Peptide 1
   Trp-Leu-Phe = Peptide 2
   Trp-Leu-Try = Peptide 3
   Trp-Leu-Arg = Peptide 4
   Trp-Leu-His = Peptide 5
   Phe-Leu-Phe = Peptide 6
   Phe-Leu-Try = Peptide 7
   Phe-Leu-Arg = Peptide 8
3. Collection of Sequences coupled to Hydroxamate:
   Phe-Leu-His = Peptide 1
   Trp-Leu-Phe = Peptide 2
   Trp-Leu-Try = Peptide 3
   Trp-Leu-Arg = Peptide 4
   Trp-Leu-His = Peptide 5
   Phe-Leu-Phe = Peptide 6
   Phe-Leu-Try = Peptide 7
   Phe-Leu-Arg = Peptide 8

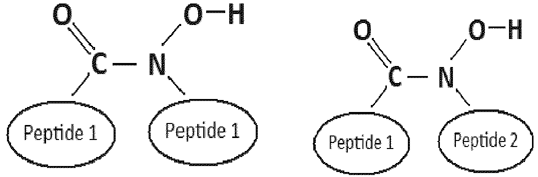

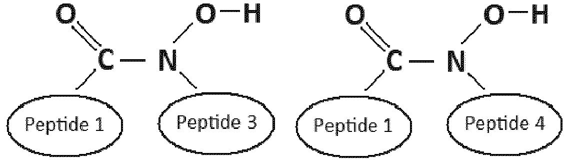

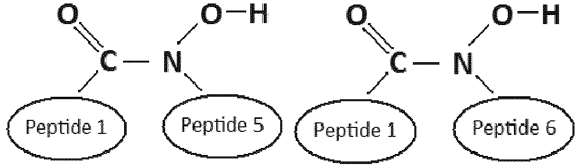

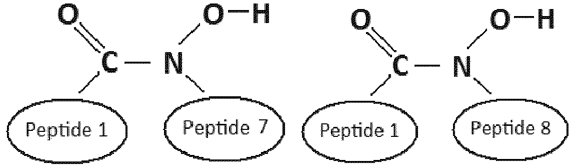

The present invention will now be described with reference to the following example, which is provided for the purpose of illustration and not intended to be construed as being limiting on the present invention.

### Example

A clinical study was carried out to evaluate the potential prophylactic and/or therapeutic properties of plasma from camels vaccinated with *Bothrops jararaca* venom when employed in a carbohydrate overload model of acute laminitis in horses.

### Protocol for Inoculation of Camels

Minimum of 3 healthy adult Dromedary Camels, which have been inspected by a Veterinarian and certified as being of good-health - castrated Males or non-lactating Females, minimum of 3 years of age, approximate weight 300-500kg.

### Antigen

Supplied pre-formulated by KLM Biotechnology Ltd (25 mgs) as a peptide solution which can be solubilised (2mgs in 2 mls) prior to being suspended in the adjuvant per animal inoculation.

### Inoculation programme

Day 0 - Baseline blood taken on all camels prior to antigen inoculation. Blood should be stored and tested for antibodies to antigen later. The animals will be immunised subcutaneously (SC) delivered into four (4) sites. 0.5ml at each inoculation site - 2 both sides of the neck, 2 both sides of the rump. These locations are designed to favour antigen presentation in draining lymph nodes. The 1^{st} inoculation (Day 1) will be administered with Freund's complete adjuvant.The 2^{nd} inoculation (Day 14) will use Freund's In-Complete adjuvant.The 3^{rd} inoculation (Day 28) will use Freund's In-Complete adjuvant. At each of these inoculation dates, a blood sample for all the camels will be taken as per Day 0 prior to inoculation to test antigen antibody titre in the Camels blood.

### Plasma Harvesting

3 litres of plasma from each camel on day 14 and day 28. Plasma needs to be filter sterilised and stored at -20'C.

In January 2015, blood collection for harvesting of camel plasma was started and continued every 1-2 weeks until sufficient plasma (∼18 L) to treat 6 horses had been collected. Blood was collected using 500 mL blood collection bags pre-filled with 63 mL of CPDA-1 (citrate/phosphate/dextrose/adenine solution) as an anticoagulant. Each bag was equipped with a 14 ga x 1.5" needle attached to 36" of tubing. These materials were used to collect blood directly from the jugular vein(s) of each camel. The name of each donor camel as well as the date of collection was recorded directly on the bag's label. After sample collection was completed, the inlet tubing was tied in a knot to prevent blood leakage and the needle was cut off along with excess tubing above the knot. Blood bags were held under refrigeration until they were processed by centrifugation approximately 2-6 days after collection. Bags of whole blood were centrifuged at 2000 RPM in a refrigerated centrifuge at 4 °C for 10 minutes. The separated plasma was then collected through the bag's outflow tubing by squeezing the bags from the bottom up and transferring the plasma into sterile, 50 mL, screw-top centrifuge tubes. The 50 mL tubes were once again centrifuged at 2000 RPM in a refrigerated centrifuge at 4 °C for 10 minutes to separate any remaining cells. Plasma was aspirated from the tubes using a 16 ga needle and 60 mL syringe, which was subsequently used to inject the plasma into empty 300 mL or 1000 mL blood storage bags (which contained no anti-coagulant). Each bag was labelled with the approximate volume (measured with the syringe), the date of collection, and the respective donor's name. Labelled bags of plasma were stored at ∼-20°C until used.

### ELISA Kit-assay

The seroconversion efficiency of the immunised Camels will be compared in ELISA time course assays using 100 ng *Antigen* /well. The 96 well plates will be blocked with 5% non-fat milk (diluted with PBS) for 3 hours at room temperature (RT), washed in five changes of TBST (0.01 M Tris-HCI, pH 8.5; 0.15 M NaCl; 1% Tween 20) incubated in sera for 3 hours at RT, washed again in TBST and incubated in the appropriate rabbit anti-camel IgG (diluted to 1:2000 in PBS) for 2 hours at RT. The plates will then be washed and incubated in horseradish peroxidise-conjugated Goat anti-rabbit IgG (1:2000) for 2 Hours at RT and the results should be visualised by addition of substrate (0.2% 2,2/-azino-bis (2-ethylbenzthiazoline-6-sulphonic acid, pH 4 in phosphate-citrate buffer containing 0.015% hydrogen peroxide).

### Protocol for Clinical Study

### Study Objective

The objective of the study is to evaluate the ability of a proprietary biological product to prevent or ameliorate clinical signs and histopathologic changes of acute laminitis. The investigational product will be administered at different time points after implementation of an oligofructose overload model shown to cause acute laminitis in mature horses.

**Table 1. Schedule of Events**

| **Study Day** | **Activity** |
|---|---|
| Prior to Day -10 | Acquisition of nine or more healthy, mature horses (3 to 7 years) as candidates. Testing to rule out PPID or EMS. |
| -10 | Begin acclimation |
| -10 to -1 | Once Daily Health Observations |
| Once between -10 and | Physical examination, body weights; lameness examinations and radiography of both forefeet |
| -4 | |
| -3, -2, -1 | Oligofructose regimen with 1 gram/kg orally once daily added to feed |
| 0 | Pre-induction lameness exam (with video record); serum sample for baseline ELISA. Complete oligofructose regimen with 10 grams/kg administered by nasogastric tube; begin clinical observations, temperature measurement and lameness monitoring q6h until development of diarrhea. |
| 0 or 1 | Continue clinical observations, temperature and lameness evaluations q6h. All groups: start intravenous fluids upon first signs of diarrhea; PCV and TP at least q4h and regulation of fluids to maintain normal hydration. |
| As needed | (Treatment of Group 2 horses upon onset of pyrexia and/or diarrhea - prior to any Obel lameness score ≥ 1). Video record of lameness exam for any horse achieving Obel ≥1. Treatment of Group 3 horses upon achieving Obel lameness score ≥1 |
| As needed | Continue fluid therapy to maintain hydration |
| As needed | Euthanasia and lamina harvest with Obel lameness score of 3 or greater. Final observations and temperature measurement prior to euthanasia. |
| As needed | Collect serum sample for ELISA. Euthanasia and lamina harvest if Obel lameness score ≤2 for 72 hours after induction. Clinical observations, temperature measurement, lameness monitoring, and lameness video prior to euthanasia. |
| | Trial terminates upon last necropsy |

### Treatment groups

The study will be performed with three treatment groups comprised of three mature horses each.

**Table 2. Study Groups**

| **Group** | **Treatment** | **Number of Horses** |
|---|---|---|
| 1 | Carbohydrate overload induction - placebo | 3 |
| 2 | Carbohydrate overload induction - treatment at first signs of pyrexia and/or diarrhea | 3 |
| 3 | Carbohydrate overload induction - treatment upon achievement of Obel lameness score ≥1 | 3 |

### Experimental design

This is an unmasked, controlled, randomized, laboratory efficacy study conducted at a single site. Mature, healthy horses with no evidence of prior laminitic episodes will be subjected to a carbohydrate overload induction model for acute laminitis. Within 12 to 24 hours after the final induction step, subjects are expected to become febrile and develop profuse diarrhea accompanied by dehydration and shock. The dramatic physiologic disruptions induced by carbohydrate overload frequently culminate in acute laminitis. All affected animals will be treated with intravenous electrolyte solutions for volume replacement and maintenance of physiologic homeostasis (volume replacement may occur simultaneously with specific treatments in Groups 2 and 3). Three horses (Group 1) will receive 6 mL/kg bodyweight of 0.9% saline solution (placebo) immediately after onset of pyrexia and/or diarrhea. Thereafter, Group 1 horses will receive fluid therapy for dehydration and shock, but no specific treatments will be administered for laminitis. Three horses (Group 2) will receive the experimental treatment immediately after the onset of pyrexia and/or diarrhea. Three remaining horses (Group 3) will receive the experimental treatment upon achievement of an Obel lameness score ≥1.

After completion of the final induction step (designated Time Zero or T.0), rectal temperatures and heart rates will be measured, mucous membranes will be evaluated for capillary refill time (CRT), and Obel lameness scores will be assigned at 6-hour intervals. Beginning with the onset of diarrhea, intravenous fluids will be administered and packed cell volume (PCV) will be measured at 2-hr intervals. Fluid administration will be titrated to maintain hydration within 4 to 6% of normal.

The humane endpoint for this study is an Obel lameness score of "3". As soon as a score of "3" is recorded, final measurements and samples will be collected, and the horse will be promptly and humanely euthanatized. After death, both forefeet will be disarticulated and the hooves will be sectioned. Specific measurements will be recorded, and representative samples of hoof lamina will be harvested and preserved for histopathologic analysis.

Any horse that does not achieve an Obel score of "3" will be maintained on study for a maximum of 72 hours after induction. After that interval, the horse will be necropsied and its hooves processed as described previously.

### Randomization and allocation

Horses meeting the inclusion criteria (see sections 9.2 and 9.3) will be ranked by decreasing body weight. Each three consecutively-ranked horses will comprise a replicate. Within each replicate, one horse will be assigned randomly to one of the three treatment groups presented in Table 1. Each treatment group will be represented once within a replicate.

### Test animals

Horses are the target species for use of the biological product to be evaluated. This study will utilize mature, random-source, light saddle breed horses, females or neutered males, and 3 to 7 years of age at the time of induction. Candidate horses must have Obel lameness scores of "0", be sound by hoof tester examination, and exhibit no radiographic evidence of prior laminitic episodes (*i.e.,* no ventral deviation of the third phalanx). Candidate horses will be healthy, as determined by clinical health observations and physical examination during the acclimation period and prior to initiation of the carbohydrate overload induction regimen.. Pre-enrollment testing of candidates will be conducted to rule out PPID (Cushing's Disease) and Equine Metabolic Syndrome (insulin resistance). Candidates must have a body condition score of ≥3 to ≤7. Female horses may not be pregnant or lactating.

At least nine candidate horses will be received at the site to begin acclimation, and any that meet inclusion criteria will be enrolled. Additional candidates may be evaluated, but ultimately, only nine will be enrolled in the study.

Horses will be derived from the resident, facility herd or purchased from a commercial livestock vendor.

All horses will be uniquely identified by a numbered neck band and by a complete physical description in the study record.

A candidate horse will be eligible for enrollment if it meets all of the following criteria:
- It conforms to the animal description in section 8.1.1 (age, gender, class, physiologic status).
- It has no significant health abnormalities, based on historical daily clinical health observations and physical examination during acclimation prior to allocation and carbohydrate overload induction.
- It has no diagnostic evidence of concurrent PPID or EMS
- It has a Body Condition Score ≥4 and ≤7.
- It has an Obel score of "0" and a hoof tester score of "0" for both forefeet
- It has no radiographic evidence of prior laminitic episodes.
- It is tractable and cooperative with study activities
- It received no treatment during the acclimation period with corticosteroids or non-steroidal anti-inflammatory drugs (NSAIDs).

A candidate will be excluded from enrollment if:
- It does not conform to inclusion criteria.
- It exhibits complicating disease conditions that may interfere with or prevent the evaluations and analyses in this study
- It has diagnostic evidence of concurrent PPID or EMS.
- It is unsound, as determined by hoof tester or Obel lameness criteria
- It exhibits radiographic evidence of prior laminitic episodes.
- It has been treated within recent history with corticosteroids or non-steroidal, anti-inflammatory drugs
- It has a body condition score (BCS) <4 or >7. #It is fractious or uncooperative with study activities.
- It received treatment with corticosteroids or NSAIDS during the acclimation period

### Duration

Horses new to the facility will be present for at least seven days prior to the start of formal acclimation. Horses will be acclimated to the facility for at least seven days prior to initiation of the carbohydrate overload induction regimen. During the acclimation period, feed, water, housing, management, and environmental conditions will simulate those expected during the study.

### Medication and/or vaccination during acclimation period

Candidate horses may be treated with approved pharmaceutical products prior to initiation of the acclimation period, but no medicinal products may be administered to subjects from the start of acclimation until completion of the study. Certain prior treatments are proscribed, as described in Section 8.2 and Section 8.3.

If any pharmaceutical product is indicated for treatment of pain, trauma, or spontaneous medical conditions, these may be given for humane reasons, but only after consultation with, and approval by, the Clinical Investigator and/or the Sponsor Representative (see section 8.14.2). All concurrent medications or therapies will be recorded in the study file and mentioned in the final report.

### Masking of study

Masking will not be implemented in the study. Testing personnel will be aware of treatment group assignments for the various animals.

Similarly, the veterinary histopathologist will be aware of group assignments when examining tissues, consistent with a consensus statement by the Society for Toxicologic Pathology (Crissman et al. Best Practices Guideline: Toxicologic Histopathology. Toxicologic Pathology 32:126-131, 2004).

### Housing enclosures

All horses acquired for the study will be housed in individual stalls measuring ∼4 m X ∼4 m floor area X ∼1.7 m high. Stalls will be constructed of portable metal panels. Flooring will be concrete, covered by rubber mats and bedded with pine or hardwood sawdust and shavings.

Horses will remain in their assigned stalls continuously, unless removed temporarily to facilitate cleaning activities or to conduct protocol activities (e.g., body weights, lameness examinations). Each stall is equipped with feeding and watering equipment, and feces are removed from the stalls daily. Soiled bedding is replaced as necessary, usually about once weekly during acclimation, and at least daily during the overload induction phase. Facility details will be described and documented in the study record.

Overhead incandescent lighting is available to provide illumination during late p.m. and early a.m. activities.

The equine housing facility is under roof, but subjects are otherwise exposed to ambient environmental conditions. Climatic conditions (minimum and maximum temperature and relative humidity) will be monitored electronically on a constant basis, and daily minima and maxima will be recorded manually on a data capture form customized for the specific study.

Each stall is equipped with a combination concentrate/hay feeder designed to offer both dietary components simultaneously. Feeders are checked daily and cleaned if necessary.

Facility water is supplied by a local utility. Fresh water is available *ad libitum,* supplied in two buckets ≥16 L in volume. Buckets are cleaned once daily and filled at least twice daily.

### Feeding

Horses will be offered a commercial horse concentrate (Co-Op #327; 11% protein) in quantities comprising 0.5% of body weight daily, divided into equal portions and offered a.m. and p.m. A feed label will be added to the study record. Horses will also be provided with grass hay at 1.5% of body weight daily, also divided into equal portions offered a.m. and p.m.

### Physical examinations

A qualified veterinarian will conduct a physical examination during the week of acclimation (between Days -10 and -4). The examination will evaluate the physiological status of each animal by systems, including rectal temperature, eyes, cardiovascular system, respiratory system, gastrointestinal and genitourinary systems, skin and hair coat, neurologic and musculoskeletal function, and overall physical condition. Findings for individual horses will be recorded on the *Physical Examination Record.*

A Body Condition Score (BCS) will be assigned to each candidate during the physical examination. Scores will range from 1 to 9, and are based on the Henneke system (Henneke et al. Relationship between condition score, physical measurements and body fat percentage in mares. Equine Veterinary Journal 15:371-372, 1983).

### Body weight

Each candidate will be weighed once between Days -10 to -4. Relevant body weights will be used to calculate appropriate quantities of oligofructose for the carbohydrate overload induction model.

Body weights will be measured with a scale that has been certified by a commercial service within 6 months before the start of the study. Prior to weighing the first animal, and again after weighing the last animal, the accuracy of the scale will be verified with standard weights ranging from 45.4 kg (100 lbs) to 364 kg (800 lbs). Body weights will be measured to the nearest kg and recorded on the *Body Weight Record.*

### Lameness examinations

Prior to enrollment, each horse will be assessed for lameness, as described in facility SOP LAM-FD-2.2. Each horse will be assessed at a walk and at a trot (if possible) to assign baseline Obel scores for each forefoot.

Guidelines for Obel lameness scoring are as follows:
Grade 0: No lameness observed at a walk or trot, even on hard surfaces.
Grade 1: The horse may alternately lift its feet, but no lameness is observable at a walk. The horse may have a short, stilted gait when trotting in a straight line on a hard surface, and turns carefully at a walk.
Grade 2: Moves with a stiff gait at the walk. The horse may have a short, stilted gait at a trot on a hard surface. Turns with great difficulty. A foot can be lifted off the ground without great difficulty.
Grade 3: Reluctant to move at a walk on any surface. It is difficult to lift a limb. The horse may be almost non-weight bearing on one limb.
Grade 4: The animal will not move, and is particularly reluctant to move from a soft to a hard surface. It is almost impossible to lift a limb.

Prior to enrollment, horses will be evaluated for foot pain using hoof testers. The hoof testers will be applied in a systemic manner to the entire sole, frog region and hoof wall to test for sensitivity/pain. A hoof tester score of "0" for both forefeet is required to be eligible for enrollment.

Guidelines for hoof tester score are as follows:
0 = No pain
1 = Mild pain is noted
2 = Moderate pain is noted
3 = Highly reactive to pain
4 = Unable to lift leg

Prior to enrollment, lateral radiographs of both forefeet of each horse will be recorded and examined for evidence of prior laminitis, defined as ventral rotation of the third phalanx (P3) in the lateral view. A written interpretation of each horse's radiographs will be included in the study record.

### Clinical observations

Clinical health observations will be recorded once daily from Day -10 to the final day of enrollment. The parameters to be observed include general health, appetite, attitude and fecal consistency (*Daily Health Observation Record*). Findings will be recorded as "normal" or "abnormal", with further characterization in the study record of any abnormal observation.

At ∼6-hour intervals (±30 minutes) after administration of the final step of the oligofructose model, general health observations will be conducted, along with measurement of rectal temperature, heart rate, and assessment of capillary refill time. Observations will be recorded on Data Capture Forms specifically created for the study, and abnormal observations will be further characterized in the study record.

Beginning when diarrhea is observed, a venous blood sample will be collected every 2 hours for measurement of packed cell volume and total protein concentration. Heart rate, CRT, and total protein concentration will be used to assess dehydration as per ETCR SOP LAM-FD-1.x. Fluid therapy will be initiated at the discretion of the veterinarian or when percentage dehydration achieves 6% or greater.

After administration of the experimental treatment (Groups 2 and 3), any abnormal health observations will qualify as Adverse Events (AE). Within 24 hours after detection of a serious AE, the Clinical Investigator will report the AE to the Sponsor Monitor, and the event will be documented on the *Adverse Event Record.* The *Adverse Event Record* will categorize the severity of the abnormal observation, and the reporting veterinarian will speculate as to the relationship of the AE to experimental treatment as follows:

**Table 3. Magnitude of Adverse Event and Relationship to Experimental Treatment**

| **Magnitude of Adverse Event** | |
|---|---|
| Score | Description |
| 1 = Mild | • Little or no discomfort. |
| | • Signs intermittent or continuous. |
| | • Baseline functions unhindered. |
| | • Not significantly hazardous to overall health. |
| | • Drug therapy and/or clinical procedure not necessary. |
| 2 = Moderate | • Some discomfort. |
| | • Signs intermittent or continuous. |
| | • Baseline functions moderately hindered. |
| | • Not significantly hazardous to overall health. |
| | • Drug therapy and/or clinical procedure may be necessary. |
| 3 = Severe | • Severe discomfort. |
| | • Signs intermittent or continuous. |
| | • Baseline functions severely hindered or prevented. |
| | • Significantly hazardous to overall health. |
| | • Drug therapy and/or clinical procedure imperative. |

| **Relationship of AE to Experimental Treatment** | |
|---|---|
| Score | Description |
| 1 = Unknown | Unknown |
| 2 = Unlikely | Unlikely since AE is clearly pre-existing or caused by specific extraneous event, with no other causative factor evident. |
| 3 = Possible | Possible based on type, time course, and relationship of AE to dosing and external events. |
| 4 = Probable | Probable based on type, time course, and relationship of AE to dosing and external events. |

### Feed and Water Consumption

Feed and hay will be provided twice daily in weighed quantities. Appetite will be characterized as:
0- consumed <25% of hay/grain
1- consumed 25-75% of hay/grain
2- consumed 75-100% of hay/grain.

Water will be provided in two, 16-L buckets per horse. Water consumption will be measured in 1/8 bucket (*i.e.,* 2-L) increments and recorded twice daily prior to refilling of the respective water buckets.

### Analytical Methods

The oligofructose overload model of inducing laminitis frequently causes severe diarrhea, so hydration will be monitored beginning at onset of diarrhea and repeated thereafter at ∼2-hr intervals for measurement of packed cell volume (PCV; hematocrit). Packed Cell Volume will be measured by methods described in facility SOP GN-LB-11.3.

Total protein will be measured by examining the plasma portion of a blood column in the microhematocrit tubes described in section 8.13.1. Total protein will be measured with an optical refractometer, as described in SOP GN-LB-11.3. The total protein concentration will be captured in the study record for every interval at which a blood sample is collected for measurement of PCV.

A serum sample (marbled red top tube; 9.5 mL draw) will be collected from each enrolled horse on Day 0 prior to induction, and again just prior to euthanasia. Serum will be harvested from each sample and stored frozen. Serum samples will be shipped to an external laboratory for measurement of camelid antibodies by a proprietary ELISA. Methods and results will be described in a separate report to be prepared by the analytical laboratory.

### Removal of subject(s) from the study

This protocol seeks to balance the need to generate relevant efficacy data with humane considerations. As such, horses experiencing adverse events, whether or not related to the test article, may receive veterinary care as medically appropriate and under the parameters described above.

A participating horse may be removed from the study if it is determined that:
- It is uncooperative with study procedures.
- It encounters a serious adverse reaction, injury, or illness necessitating treatment with contraindicated, concomitant medications (see section above) or dictating immediate removal for humane reasons.
- It dies spontaneously or is euthanatized.

A horse will be removed from the study if any of the indicated removal criteria apply. The Clinical Investigator will consult with the Sponsor whenever possible prior to removing a horse from the study. However, the final decision whether to remove a horse from the study will rest with the Clinical Investigator. The Clinical Investigator will document the horse's identity, the date of the removal, the reason for the removal, and the fate of the animal. Data generated by removed animals up to the point of removal will be included in study analyses.

Horses withdrawn from the study after being dosed with investigational product will be subject to euthanasia and necropsy, as described in below.

### Induction of acute laminitis

### Pre-induction dietary regimen

On Days -3 to -1, one gram of oligofructose (BENEO® 95; Orafti) will be added to the a.m. basal ration of each scheduled candidate. Preparation and administration will be documented in the study record.

### Induction of acute laminitis

On Day 0, 10 grams of oligofructose (BENEO 95; Orafti) per kg of body weight will be dissolved in ≥4 liters of tepid tap water. The oligofructose solution will be administered to horses via nasogastric tube. This event will be termed "Time 0" or "T.0". Preparation and administration will be documented in the study record.

### Euthanasia

Horses will be sedated with xylazine or medetomidine and humanely euthanatized in compliance with recommendations of the 2013 AVMA Guidelines for Euthanasia. Specifically, horses will be dropped with a captive bolt stunner and exsanguinated, as described in facility SOP EQ-NX-1.4. Relevant procedures will be documented on the *Equine Euthanasia Record.*

### Investigational Product

### Chemical name

Hyperimmunized camel plasma

### Trade name

PTP-100 (KLM's Trade name for product)

### Active ingredients

Albumin and globulin fractions of dromedary camel blood. Putatively contains specific camelid antibodies (IgG) to venom of *Bothrops jararaca.*

### Dosing form

The investigational product will be thawed plasma in polyethylene storage bags intended for intravenous infusion.

### Dose to be tested

The recommended dose to be tested is 6 mL of thawed plasma per kg body weight.

### Derivation site

East Tennessee Clinical Research, Inc.

80 Copper Ridge Farm Rd.

Rockwood, TN 37854

U.S.A.

### Certificate of Analysis

No certificate of analysis will be issued for the proprietary products, but serum samples will be collected from each vaccinated camel at each plasma collection time point. Serum samples will be analyzed for specific antibodies with a proprietary ELISA test.

### Lot No.

Lot numbers will be assigned to each collection of plasma. Lots will be coded to indicate the donor camel and date of collection. Depending on ELISA results, plasma samples from various animals might be pooled prior to administration to enrolled horses.

### Expiration date

None will be assigned for this investigational product. The stability of the investigational product is unknown, but is assumed to be optimized by storage in frozen conditions.

### Storage during study

During the study, the investigational product will be stored in a freezer and maintained at temperatures <-20°C. Storage conditions will be monitored regularly and recorded.

### Investigational product administration

### Timing of administration

Horses assigned to Group 1 will receive 0.9% sodium chloride solution upon the first observation of pyrexia (rectal temperature ≥102.0°F) and/or diarrhea.

Horses assigned to Group 2 will receive their assigned dose of investigational product upon the first observation of pyrexia (rectal temperature ≥102.0°F) and/or diarrhea.

Horses assigned to Group 3 will receive their assigned dose of investigational product upon the first observation of an Obel lameness score ≥"1".

Horses in all three groups will be treated with intravenous electrolyte solutions in sufficient quantities to maintain hydration within 4% - 6% of normal.

### Route and method of administration

Each assigned dose of investigational product will consist of 6 mL of thawed, hyperimmune camel plasma warmed to body temperature (∼100°F) per kg of bodyweight. Each complete dose will be administered by constant intravenous infusion over an interval of approximately 1 hour or longer.

### SPECIFICATION OF VARIABLES

### Lameness Assessment

Obel lameness scores will be assessed at 6-hour intervals (T.4, T.8, etc.) beginning after the final step of induction (T.0). The pre-induction lameness examination will be captured on video, as will subsequent examinations once an individual horse achieves an Obel lameness score ≥1.

### Laminar Measurements for Rotation of Distal Phalanx

Once death has been confirmed, both forefeet will be removed and the hooves will be processed by the methods of Pollitt C.C. (1996) (Basement Membrane Pathology: a feature of acute equine laminitis. Equine Veterinary Journal 28(1):38-46)) and consistent with ETCR SOP LAM-FD-5.2. Measurements will be taken from a midline sagittal section of both forefeet. The distance between the anterior edge of the distal phalanx (P3) and the posterior edge of the white line will be measured at two points, along lines constructed perpendicular to the anterior surface of P3. The first measurement will be taken at the distal edge of the coronary band, and a second at the distal extremity of P3. Methods and equipment will be described in the study record. Distal measurements that are greater than proximal dimensions may indicate laminar separation and palmar rotation of the distal phalanx away from the hoof wall. Measurements will be documented in the study record.

### Laminar Tissue Samples for Histopathology

Using the methods of Pollitt (1996), four 2-cm x 2-cm laminar tissue blocks will be collected from each hoof, if possible, and preserved. Specific procedures are defined in facility SOP LAM-FD-5.2.

Hoof samples will be collected and preserved in 10% neutral buffered formalin for 12 to 24 hours, and then transferred to 70% alcohol (per SOP LAM-FD-5.2). Two blocks of preserved samples per hoof will be shipped to a histopathology laboratory (HistoTechniques, Powell, OH) for sectioning and mounting. One set will be stained for analysis by the study histopathologist and a duplicate set will be left unstained for possible immunohistochemical or other types of analysis. A duplicate set of blocks (*i.e.,* two per hoof) will be retained at the testing facility.

### DATA ANALYSIS

### Experimental unit

The experimental unit is the individual horse. Statistical analysis will be done at the discretion of the Sponsor. Clinically valid cases will be included in the evaluation of treatment success and the effectiveness outcomes. All horses that received an injection of the investigational product will be included in the analysis of the safety outcomes.

### COLLECTION AND RETENTION OF SOURCE DATA

Raw data will be collected, recorded, archived, and retained according to current versions of test facility SOPs, this protocol, and applicable regulatory requirements. Hand-written data will be recorded per facility SOPs. All visits and telephone conversations relative to the study will be documented and all correspondence (including Email messages) will be filed with the study record. All original data collected and records generated, will be appended to the final study report.

All original data collected, records generated, non-labile specimens obtained, and final reports written in connection with the study will be returned to the Sponsor. Certified copies of all raw data, records, and reports will be archived at the test facility for at least five years following completion of the study.

### RESULTS

As outlined above, nine healthy mature horses between 4 and 9 years of age were evaluated for enrolment in the clinical study. Evaluations included physical examination, a lameness exam including evaluation for foot soreness with a hoof tester, and radiography of both forefeet to ensure that no prior episodes of laminitis had resulted in rotation of the third phalanx. Preliminary examinations were deemed normal for all candidates. Eligible candidates underwent a formal acclimation period of 10 days, during which feed, water, housing and management were identical to conditions of the actual study. On Days -3, -2, and -1, oligofructose was added to the a.m. grain ration of each candidate at a dosage of 1 gram per kg body weight.

Prior to induction on Day 0, a baseline lameness exam was repeated and captured on video. Immediately thereafter, the carbohydrate overload model was completed by administration of 10 grams of oligofructose/kg body weight mixed with tepid water and administered by nasogastric tube. This model causes an overgrowth of Gram positive bacteria in the large bowel, consistently resulting in diarrhoea, acidosis, and volume depletion. Approximately 80% or more of horses subjected to this model also develop acute laminitis within 24 to 36 hours.

Horses were ranked by decreasing magnitude of body weight. Each three consecutively-ranked horses comprised a replicate, and each horse within a replicate was allocated randomly to one of the following three treatment groups:
Group 1 - intravenous administration of placebo (3 litres of 0.9% saline solution) upon the first evidence of diarrhoea with or without an elevated temperature;
Group 2 - intravenous administration of plasma (6mL/kg) from camels with antibodies to *B. Jararaca* venom upon first evidence of diarrhoea with or without an elevated temperature;
Group 3 - intravenous administration of plasma (6mL/kg) from camels with antibodies to *B. Jararaca* venom upon first evidence of lameness, characterized as an Obel lameness score of "1" Or greater.

Hereafter, the various treatment groups may be referred to as "control", "prophylaxis", or "treatment", respectively. The demographic information and treatment allocations for all enrolled horses are presented in Table 1.

**Table 4. Demographic and treatment allocation of horses**

| **Treatment Group** | **I.D. No.** | **Age (yrs)** | **Sex** |
|---|---|---|---|
| Group 1 Placebo ("control") | 735 | 8 | F |
| | 736 | 9 | F |
| | 763 | 4 | F |
| Group 2 Plasma at diarrhoea ("prophylaxis") | 729 | 8 | F |
| | 611 | 9 | F |
| | 789 | 6 | F |
| Group 3 Plasma at lameness ("treatment") | 728 | 7 | MC |
| | 572 | 9 | F |
| | 772 | 6 | MC |

| | | | |
|---|---|---|---|
| MC = male castrate (gelding) | | | |

At regular, 6-hr intervals after induction, the general health of each animal was assessed, a lameness exam was repeated, and rectal temperature was monitored. Once diarrhoea had begun, each horse's hydration status was monitored at 2- to 4-hr intervals by assessing its packed cell volume (PCV; hematocrit) and plasma total protein concentration. Intravenous fluids (lactated Ringer's or 0.9% sodium chloride solution) were administered in sufficient volumes to maintain a target PCV of ≤40%. No other medications were administered during the test period. As indicated earlier, placebo or plasma were administered to horses enrolled in Groups 1 or 2 at the first signs of diarrhoea. Treatment of Group 3 horses were initiated immediately after the first lameness exam that exhibited an Obel score of "1" or greater. An additional, time-stamped video record was made of the lameness exams of any horse with an Obel score of "1" or greater.

Intravenous fluids were continued until the animal was able to maintain a fairly consistent hydration status. Individual horses in this study required between 0 and 63 litres of I.V fluids to maintain homeostasis. The single horse (#772) that required no fluid support was allocated to Group 3 (treatment).

Any horse that achieved an Obel lameness score of "3" was promptly and humanely euthanatized. All surviving horses, regardless of Obel score, were sacrificed at 72 hours after induction. After death, both forelimbs were removed and the horses were sectioned and processed for recovery of blocks of laminar tissue for histopathologic examination.

Preliminary findings of the study are presented in tabular form in Table 2.

**Table 5. Lameness progression patterns and time of euthanasia of horses**

| **Treatment Group** | **I.D. No.** | **Time of Euth. (hrs)** | **Obel Score at examination intervals** | | | | | | | | | | | | |
|---|---|---|---|---|---|---|---|---|---|---|---|---|---|---|---|
| | | | **0** | **6** | **12** | **18** | **24** | **30** | **36** | **42** | **48** | **54** | **60** | **66** | **72** |
| Group 1 "control" | 735 | 30 | 0 | 0 | 0 | 0 | 2 | 3 | | | | | | | |
| | 736 | 24 | 0 | 0 | 0 | 2 | 3 | | | | | | | | |
| | 763 | 30 | 0 | 0 | 0 | 0 | 1 | 3 | | | | | | | |
| Group 2 "prophylaxis " | 729 | 72 | 0 | 0 | 0 | 0 | 0 | 1 | 1 | 1 | 1 | 0 | 0 | 0 | 0 |
| | 611 | 48 | 0 | 0 | 0 | 0 | 0 | 0 | 1 | 2 | 3 | | | | |
| | 789 | 72 | 0 | 0 | 0 | 0 | 1 | 0 | 0 | 0 | 0 | 0 | 0 | 0 | 0 |
| | | | | | | | | | | | | | | | |
| Group 3 "treatment" | 728 | 72 | 0 | 0 | 0 | 0 | 1 | 2 | 2 | 2 | 2 | 1 | 1 | 1 | 1 |
| | 572 | 42 | 0 | 0 | 0 | 1 | 1 | 2 | 2 | 3 | | | | | |
| | 772 | 54 | 0 | 0 | 0 | 0 | 1 | 1 | 2 | 2 | 2 | 3 | | | |

| | | | | | | | | | | | | | | | |
|---|---|---|---|---|---|---|---|---|---|---|---|---|---|---|---|
| Empty boxes = no subsequent readings because the horse had achieved a score of "3" and had been euthanatized. | | | | | | | | | | | | | | | |

### Discussion

All nine horses enrolled in the study developed diarrhoea and varying degrees of volume depletion. More importantly, all nine horses exhibited at least one elevated lameness score after induction, establishing an incidence of 100% for sign of acute laminitis attributable to the model. The mean time for induction to achievement of an Ocel lameness score of"3", and subsequent euthanasia were:
Group 1 - 28 hours
Group 2 - 64 hours
Group 3 - 56 hours

All Group 1 horses developed Grade 3 lameness and were euthanized by 30 hours after induction. This group (control) represents the progression of acute laminitis in naturally-occurring cases in the absence of any effective treatment.

One 1 horse in Group 2 (prophylaxis) developed Grade 3 lameness and was euthanatized at 48 hours post-induction. However, this horse remained free of clinical signs for 30 hours after induction, but developed progressively worsening lameness beginning at 36 hours. The remaining horses in Group 2 developed temporary signs of mild lameness (Obel Grade 1), but returned to normal for at least 24 hours prior to euthanasia.

Two of three horses in Group 3 (treatment) developed Grade 3 lameness and were euthanized at 42 and 54 hours post-induction. The single surviving horse developed more severe lameness (Obel Grade 2) than any horse in the prophylaxis group, but was showing definite signs of recovery (Obel Grade 1) prior to euthanasia.

All horses that received the unrefined plasma product developed hives and pruritus (itching) while the product was being administered intravenously, and most continued to display those signs for several hours after treatment. These adverse signs were temporary, however, and had disappeared from all treated animals within 6 to 12 hours post-treatment. It is hypothesised that the hives are attributable to an allergic reaction, probably a minor graft vs. host reaction associated with the presence of various unclassified antibodies in the unrefined plasma. One horse (#772) in Group 3 developed temporary signs of restlessness, agitation, and spontaneous muscle twitching. These signs were attributed to hyperkalemia (elevated plasma potassium level). This episode was treated successfully by administration of oral glucose and a small volume (0.5L) of saline solution administered intravenously. This adverse event was attributed to a general electrolyte imbalance associated with diarrhoea, rather than to a specific response to plasma administration.

### Conclusion

The most favourable clinical response was observed in Group 2 horses, i.e., those administered camel plasma at the first signs of a clinical condition (diarrhoea from carbohydrate overload) that commonly results in acute laminitis. It is considered that the single Group 2 horse that developed lameness beginning at 36 hours post-induction did not represent a treatment failure, but rather this incident suggests that the dose of antibodies administered was insufficient to block all of the pathophysiologic mechanisms of acute laminitis. Specifically, it suggests that the optimal course of treatment might require two or more doses of plasma, administered at an interval that remains to be investigated.

The clinical responses observed in Group 3 (treatment) horses were obvious, but less complete than when the product was used prophylactically. It may be that the clinical responses of this group would also be improved by multiple doses of plasma administered at target intervals after exposure to a predisposing event that commonly results in acute laminitis.

All documents referred to in this specification are herein incorporated by reference. Various modifications and variations to the described embodiments of the inventions will be apparent to those skilled in the art without departing from the scope of the invention.

### Laminitis Study Results with anti hemorrhage peptide LTNF :

This communication is a brief report on the results obtained from the recent clinical study to evaluate the potential prophylactic and/or therapeutic properties of KLM's proprietary application of a 15 amino acid sequence termed LTRF in saline when employed in a carbohydrate overload model of acute laminitis in horses.

In compliance with the methods outlined in protocol KLM-15-02, nine healthy, mature horses between 4 and 9 years of age were evaluated for enrollment in the clinical study. Evaluations included physical examination, a lameness exam including evaluation for foot soreness with a hoof tester, and radiography of both forefeet to ensure that no prior episodes of laminitis had resulted in rotation of the third phalanx. Preliminary examinations were deemed normal for all candidates. Eligible candidates underwent a formal acclimation period of 10 days, during which feed, water, housing and management were identical to conditions of the actual study. On Days -3, -2, and -1, oligofructose was added to the a.m. grain ration of each candidate at a dosage of 1 gram per kg body weight.

Prior to induction on Day 0, a baseline lameness exam was repeated and captured on video. Immediately thereafter, the carbohydrate overload model was completed by administration of 10 grams of oligofructose/kg body weight mixed with tepid water and administered by nasogastric tube. This model causes an overgrowth of Gram positive bacteria in the large bowel, consistently resulting in diarrhea, acidosis, and volume depletion. Approximately 80% or more of horses subjected to this model also develop acute laminitis within 24 to 36 hours.

Horses were ranked by decreasing magnitude of body weight. Each three consecutively-ranked horses comprised a replicate, and each horse within a replicate was allocated randomly to one of the following three treatment groups:
Group 1 - intravenous administration of placebo (3 liters of 0.9% saline solution) upon the first evidence of diarrhea with or without an elevated temperature;
Group 2 - intravenous administration of the LTNF 15 amino -acid sequence in saline , three doses of LTNF peptide were used /Horse No 1 received 2 grams of LTNF peptide immediately following administration of the final dose of Oligofructose /Horse No 2 received 4 grams of LTNF peptide again immediately following administration of the final dose of Oligofructose. / Horse No 3 received 8 grams of LTNF peptide again immediately following administration of the final dose of Oligofructose.
Group 3 - intravenous administration of the LTNF peptide in three differing doses to horses. Horse No1 received 2 grams of LTNF .Horse No 2 received 4 grams of LTNF peptide and Horse No 3 received 8 grams of LTNF peptide suspended in saline upon the first evidence of lameness, characterized as an Obel lameness score of "1" or greater.

Hereafter, the laminitis treatment groups may be referred to as "control", "prophylaxis", or "treatment", respectively. The demographic information and treatment allocations for all enrolled horses are presented in Table 1.

**Table 1. Demographics and treatment allocation of horses enrolled in KLM-15-02**

| **Treatment Group** | **I.D. No.** | **Age (yrs)** | **Sex** |
|---|---|---|---|
| **Group 1 Placebo ("control")** | 961 | 7 | F |
| | 962 | 7 | F |
| | 963 | 8 | F |
| **Group 2 Three differing doses of peptide LTNF initiation("prophylaxis")** | 864 | 6 | F |
| | 872 | 8 | F |
| | 866 | 6 | F |
| **Group 3 Three differing doses of peptide LTNF at lameness ("treatment")** | 446 | 5 | F |
| | 432 | 8 | F |
| | 427 | 7 | F |

At regular, 6-hr intervals after induction, the general health of each horse was assessed, a lameness exam was repeated, and rectal temperature was monitored. Once diarrhea had begun, each horse's hydration status was monitored at 2- to 4-hr intervals by assessing its packed cell volume (PCV; hematocrit) and plasma total protein concentration. Intravenous fluids (lactated Ringer's or 0.9% sodium chloride solution) were administered in sufficient volumes to maintain a target PCV of <40%. No other medications were administered during the test period. As indicated earlier, placebo or LTNF peptide in saline were administered to horses enrolled in Groups 2 upon study commencement Treatment of Group 3 horses was initiated immediately after the first lameness exam that exhibited an Obel score of "1" or greater.

Intravenous fluids were continued until the animal was able to maintain a fairly consistent hydration status. Individual horses in this study required between 23 and 471iters of I.V. fluids to maintain homeostasis.

Any horse that achieved an Obel lameness score of "3" was promptly and humanely euthanatized. All surviving horses, regardless of Obel score, were sacrificed at 72 hours after induction. After death, both forelimbs were removed and the hooves were sectioned and processed for recovery of blocks of laminar tissue for histopathologic examination.

### Results

Preliminary findings of the study are presented in tabular form in Table 2.

**Table 2. Lameness progression patterns and time of euthanasia of 9 horses enrolled in KLM-15-02**

| **Treatment Group** | **I.D. No.** | **Time of Euth. (hrs)** | **Obel score at examination intervals** | | | | | | | | | | | | |
|---|---|---|---|---|---|---|---|---|---|---|---|---|---|---|---|
| | | | **0** | **6** | **12** | **18** | **24** | **30** | **36** | **42** | **48** | **54** | **60** | **66** | **72** |
| **Group 1 "control"** | 961 | 30 | 0 | 0 | 0 | 0 | 2 | 3 | | | | | | | |
| | 962 | 24 | 0 | 0 | 0 | 2 | 3 | | | | | | | | |
| | 963 | 30 | 0 | 0 | 0 | 0 | 2 | 3 | | | | | | | |
| | | | | | | | | | | | | | | | |
| **Group 2 "prophylaxis"** | 864 | 72 | 0 | 0 | 0 | 0 | 0 | 1 | 1 | 2 | 2 | 0 | 0 | 0 | 0 |
| | 872 | 72 | 0 | 0 | 0 | 0 | 0 | 0 | 1 | 2 | 2 | | | | |
| | 866 | 72 | 0 | 0 | 0 | 0 | 0 | 0 | 0 | 0 | 0 | 0 | 0 | 0 | 0 |
| | | | | | | | | | | | | | | | |
| **Group 3 "treatment"** | 446 | 72 | 0 | 0 | 0 | 0 | 1 | 2 | 2 | 2 | 2 | 1 | 1 | 1 | 1 |
| | 432 | 42 | 0 | 0 | 0 | 1 | 1 | 1 | 2 | 3 | | | | | |
| | 427 | 54 | 0 | 0 | 0 | 0 | 1 | 1 | 2 | 2 | 2 | 3 | | | |

Intravenous fluids were continued until the animal was able to maintain a fairly consistent hydration status. Individual horses in this study required between 23 and 471iters of I.V. fluids to maintain homeostasis.

### Discussion

All nine horses enrolled in the study developed diarrhea and varying degrees of volume depletion. More importantly, all nine horses exhibited at least one elevated lameness score after induction, establishing an incidence of 100% for signs of acute laminitis attributable to the model. The mean times from induction to achievement of an Obel lameness score of "3", and subsequent euthanasia were:
Group One 24 to 30hours
Group Two- 72Hours
Group Three -42-72 hours

All Group 1 horses developed a Grade 3 lameness and were euthanatized by 30 hours after induction. This group (control) represents the progression of acute laminitis in naturally-occurring cases in the absence of any effective treatment.

Horses in Group 2 (prophylactic) developed temporary signs of mild lameness (Obel Grade 1), but returned to normal prior to euthanasia.

Two of three horses in Group 3 (treatment) developed a Grade 3 lameness and were euthanatized at 42 and 54 hours post-induction. The single surviving horse developed lameness, but was showing definite signs of recovery (Obel Grade 1) prior to euthanasia.

All six of the horses that received intravenous peptide LTNF in differing doses exhibited lower lameness scores on average than the control horses. Furthermore, all survived for a longer period than any of the control horses before developing a Grade 3 lameness, which occurred in two Group 3. respectively.

All horses that received the intravenous LTNF peptide demonstrated no signs of immune reaction or toxicity.

### Conclusions

The administration of the anti-hemorrhagic peptide LTNF to the prophylactic group at all dose levels produced very dramatic results that require confirmation histopathology.slide review.

### Statements

The invention will now be described by the follow set of numbered statements:
Statement 1. A method for treating or preventing laminitis in an ungulate in need thereof, the method comprising administering to the ungulate a therapeutically or prophylactically effective amount of a camelid protease inhibitor.
Statement 2. The method of statement 1 wherein the ungulate is selected from the group consisting of equidae, bovinae, suidae, deer, ovis and capra.
Statement 3. The method of statement 2 wherein the ungulate is a horse.
Statement 4. The method of any preceding statement wherein the camelid protease inhibitor is an inhibitor of equine metalloproteinases and equine serine proteases.
Statement 5. The method of any preceding statement wherein the camelid protease inhibitor occurs naturally in blood from healthy camelid or is a recombinant form thereof.
Statement 6. The method of any one of statements 1 to 4 wherein the camelid protease inhibitor is generated by inoculating camelid with purified equine metalloproteinase enzymes and serine proteases.
Statement 7. The method of any one of statements 1 to 4 wherein the camelid protease inhibitor is generated by inoculating camelid with snake venom metalloproteinases.
Statement 8. The method of statement 7 wherein the snake venom metalloproteinases are obtained from *Bothrops jararaca.*
Statement 9. The method of any preceding statement wherein the camelid protease inhibitor is a homodimer antibody or an antigen binding fragment of same.
Statement 10. The method of preceding statement wherein the camelid protease camelid protease inhibitor is administered simultaneously, separately or sequentially to a peptide comprising SEQ ID NO:1 or SEQ ID NO:2.
Statement 11. A composition comprising a camelid protease inhibitor which inhibits equine metalloproteinases and equine serine proteases wherein the camelid protease inhibitor is isolated from camelid blood or is a recombinant form thereof.
Statement 12. A composition comprising a camelid protease inhibitor which inhibits equine metalloproteinases and equine serine proteases wherein the camelid protease inhibitor is generated by inoculating camelid with purified equine metalloproteinase enzymes and serine proteases.
Statement 13. A composition comprising a camelid protease inhibitor which inhibits equine metalloproteinases and equine serine proteases wherein the camelid protease inhibitor is generated by inoculating camelid with snake venom metalloproteinases.
Statement 14. The composition of statement 13 wherein the snake venom metalloproteinases are obtained from *Bothrops jararaca.*
Statement 15. The composition of any one of statements 11 to 14 wherein the camelid protease inhibitor is a homodimer antibody or an antigen binding fragment of same.
Statement 16. The composition of any one of statements 11 to 15 wherein the composition comprises a peptide comprising SEQ ID NO:1 or SEQ ID NO:2 both peptides inhibit equine metalloproteases and equine serine proteases.
Statement 17. A composition of any one of statements 11 to 16 for use as a medicament.
Statement 18. A composition of any one of statements 11 to 16 for use in treating or preventing laminitis in an ungulate in need thereof.
Statement 19. The composition for use of statement 18 wherein the ungulate is a horse.
Statement 20. A pharmaceutical composition comprising the composition of any one of statements 11 to 16 and at least one pharmaceutically acceptable excipient.
Statement 21. A method for generating antibodies comprising inoculating camelid with one or more proteases selected from the group consisting of equine metalloproteinase enzymes, equine serine proteases and snake venom metalloproteinases (SVMP).
Statement 22. The method of statement 21 wherein the snake venom metalloproteinases are obtained from *Bothrops jararaca.*
Statement 23. A method for at least partially purifying or enriching a camelid protease inhibitor which inhibits equine metalloproteinases and equine serine proteases, the method comprising steps of subjecting camelid serum and/or camelid plasma to one or more treatment steps selected from the group consisting of centrifugation, micro-filtration, ultra-filtration, ion-exchange chromatography, molecular sieve chromatography, affinity chromatography, reverse-phase high performance liquid chromatography and transient acidification.
Statement 24. A method for treating or preventing laminitis in an ungulate in need thereof, the method comprising administering a therapeutically or prophylactically effective amount of an anti-hemorrhagic peptide comprising SEQ ID NO:1 or SEQ ID NO:2.
Statement 25. A composition comprising a camelid protease inhibitor which inhibits equine metalloproteases and equine serine proteases wherein the camelid protease inhibitor is generated by inoculating camelids with whole snake venom.
Statement 26. A method for treating or preventing laminitis in an ungulate in need thereof, the method comprising administering to the ungulate a therapeutically or prophylactically effective amount of the peptide SEQ ID No 1 and /or SEQ ID No 2.
Statement 27. A method for treating or preventing Equine Chronic Lung disease in an ungulate in need thereof, the method comprising administrating to the ungulate a therapeutically or prophylactically effective amount of a camelid protease inhibitor and/or any of the peptides SEQ:ID No 1and/or SEQ:ID No 2
Statement 28. A method for treating or preventing Equine osteoarthritis disease in an ungulate in need thereof, the method comprising administrating to the ungulate a therapeutically or prophylactically effective amount of a camelid protease inhibitor and/or any of the peptides SEQ:ID No 1 and/or SEQ:ID No 2.
Statement 29. A method for treating or preventing equine septic joint disease in an ungulate in need thereof, the method comprising administrating to the ungulate a therapeutically or prophylactically effective amount of a camelid protease inhibitor and/or any of the peptides SEQ:ID No 1 and/or SEQ:ID No 2.
Statement 30. A method for treating or preventing equine colic disease in an ungulate in need thereof, the method comprising administrating to the ungulate a therapeutically or prophylactically effective amount of a camelid protease inhibitor and/or any of the peptides SEQ:ID No 1 and/or SEQ:ID No 2.
Statement 31. A method for treating or preventing Equine Chronic obstructive pulmonary disease in an ungulate in need thereof, the method comprising administrating to the ungulate a therapeutically or prophylactically effective amount of a camelid protease inhibitor and/or any of the peptides SEQ:ID No 1 and/or SEQ:ID No 2.
Statement 32. A method for treating or preventing equine ulcerative colitis disease in an ungulate in need thereof, the method comprising administrating to the ungulate a therapeutically or prophylactically effective amount of a camelid protease inhibitor and/or any of the peptides SEQ:ID No 1 and/or SEQ:ID No 2.
Statement 33. A method for treating or preventing Equine Crohns disease in an ungulate in need thereof, the method comprising administrating to the ungulate a therapeutically or prophylactically effective amount of a camelid protease inhibitor and/or any of the peptides SEQ:ID No 1 and/or SEQ:ID No 2.
Statement 34. A method for treating or preventing equine inflammatory bowel disease in an ungulate in need thereof, the method comprising administrating to the ungulate a therapeutically or prophylactically effective amount of a camelid protease inhibitor and/or any of the peptides SEQ:ID No 1and/or SEQ:ID No 2.

## Claims

1. A composition for use in a method of treating or preventing a disorder associated with undesirable protease activity in an ungulate in need thereof, the method comprising administering a therapeutically or prophylactically effective amount of an anti-hemorrhagic peptide, wherein the anti-hemorrhagic peptide comprises the sequence of SEQ ID NO:2.

2. The composition for use of claim 1, wherein the anti-hemorrhagic peptide comprises the sequence of SEQ ID NO:1.

3. The composition for use of claim 1 or 2, wherein the disorder is laminitis.

4. The composition for use of claim 1, 2, or 3, wherein the ungulate is a hoofed ungulate.

5. The composition for use of any one of claims 1 to 4, wherein the ungulate is selected from the group consisting of equidae, bovinae, suidae, deer, ovis and capra.

6. The composition for use of any one of claims 1 to 5, wherein the ungulate is a horse.

7. The composition for use of claim 3, wherein the laminitis is chronic laminitis.

8. The composition for use of claim 3, wherein the laminitis is acute laminitis.

9. The composition for use of any preceding claim, wherein the anti-hemorrhagic peptide is obtained from opossum serum or cotton rat, or is a recombinant form thereof.

10. The composition for use of claim 1 or 2, wherein the disorder is selected from the group comprising equine chronic lung disease, equine osteoarthritis disease, equine septic joint disease, equine colic disease, equine chronic obstructive pulmonary disease, equine joint disease, equine ulcerative colitis disease, equine Crohns disease and equine inflammatory bowel disease.


SEQUENCE LISTING

<110>  BYROCK TECHNOLOGIES LIMITED

<120>  COMPOSITIONS AND METHODS FOR TREATMENT OF DISEASES

<130>  P129081EP01

<150>  IE 2014/0274
<151>  2014-10-15

<150>  IE 2015/0034
<151>  2015-01-13

<150>  IE 2015/0183
<151>  2015-06-18

<150>  IE 2015/0270
<151>  2015-08-17

<160>  2

<170>  PatentIn version 3.5

<210>  1
<211>  15
<212>  PRT
<213>  Unknown

<220>
<223>  The anti-hemorrhagic peptide may be obtained from opossum serum
       or cotton rat, or may be a recombinant form thereof.

<400>  1

Leu Lys Ala Met Asp Pro Thr Pro Pro Leu Trp Ile Lys Thr Glu
1               5                   10                  15

<210>  2
<211>  10
<212>  PRT
<213>  Unknown

<220>
<223>  The anti-hemorrhagic peptide may be obtained from opossum serum
       or cotton rat, or may be a recombinant form thereof.

<400>  2

Leu Lys Ala Met Asp Pro Thr Pro Pro Leu
1               5                   10